# EUROPEAN PATENT APPLICATION

(11) **EP 4 502 602 A1**
(43) Date of publication of application: **05.02.2025**
(21) Application number: 23774948.6
(22) Date of filing: 22.03.2023
(51) Int. Cl.: G01N 33/53, C07K 16/18

(54) **IMMUNOLOGICAL DETECTION METHOD AND IMMUNOLOGICAL DETECTION KIT**

(30) Priority: 25.03.2022 JP 2022050823
(71) Applicant: Sekisui Medical Co., Ltd., Tokyo 103-0027 (JP)
(72) Inventor: HORIUCHI MORISHITA, Shiomi, Tokyo 103-0027 (JP); OGASAWARA, Rikako, Tokyo 103-0027 (JP); SAHARA, Ryo, Tokyo 103-0027 (JP); ASAI, Tomohide, Tokyo 103-0027 (JP); MIYAZAKI, Osamu, Tokyo 103-0027 (JP)
(74) Representative: Ter Meer Steinmeister & Partner
(86) International application number: PCT/JP2023/011204
(87) International publication number: WO 2023/182353

(57) **Abstract**

The present invention provides an immunological detection method for trimeric type I collagen N-terminal propeptide in a biological sample, which uses a first antibody that binds to a specific portion of a pro-α1 chain in the trimeric type I collagen N-terminal propeptide and a second antibody that binds to a specific portion of a pro-α2 chain in the trimeric type I collagen N-terminal propeptide. The immunological detection method is easy to handle and can specifically (selectively) measure the trimer in biological samples containing the trimer and monomer of the α-chain of PINP.

## Description

### [Technical Field]

The present invention relates to an immunological detection method and an immunological detection kit.

Priority is claimed on Japanese Patent Application No. 2022-050823, filed March 25, 2022, the contents of which are incorporated herein by reference.

### [Background Art]

Type I procollagen N-propeptide (hereinafter referred to as "P1NP" or "PINP") is a polypeptide with a molecular weight of approximately 35 kDa that is cleaved from the N-terminus when type I collagen, the main component of bone matrix protein, is produced from the precursor type I procollagen. PINP released into the blood is known to be a bone formation marker that sensitively reflects early stage of bone formation. Therefore, blood PINP concentration is useful for determining and monitoring the therapeutic effects of osteoporosis drugs such as PTH (parathyroid hormone) preparations and bisphosphonate preparations. The measurement of PINP (total PINP) sometimes results in detection of monomers as well as intact PINP as a trimer.

As type I procollagen-N-propeptide kit (generic name), for example, Procollagen Intact PINP (product name, manufactured by Fujirebio Inc., Non-Patent Document 1) and Elecsys (registered trademark) Reagent Total P1NP (product name, Roche Diagnostics, Non-Patent Document 2) are commercially available.

Patent Document 1 discloses a method and a test kit for determining the presence of amino-terminal propeptides of type I procollagen in individual persons.

Patent Literature 2 discloses an assay method for the α1-homotrimer amino-terminal propeptide which is an intact trimer of type I procollagen or the heterotrimer amino-terminal propeptide which is an intact classical type I procollagen, and an assay kit suitable for use in the assay method.

Non-Patent Literature 3 discloses that a trimeric PINP and a monomeric PINP have different clearance pathways (trimer: liver, monomer: kidney), and that patients with renal disease tend to lack ability of form clearance of the monomer and have high monomer concentrations in the blood.

Non-Patent Literature 4 discloses that a monomeric PINP reflects the degradation product of pN-collagen rather than the thermal denaturation product of the intact propeptide.

### [Citation List]

### [Patent Literature]

Patent Literature 1: Japanese Unexamined Patent Application Publication (Translation of PCT Application) No. Hei 7-504896
Patent Literature 2: Japanese Unexamined Patent Application, First Publication No. Hei 8-291197

### [Non Patent Literature]

Non-Patent Literature 1: Package insert (13th edition) of "Type I Procollagen-N-propeptide Kit Procollagen Intact PINP", Fujirebio Inc., February 2020
Non-Patent Literature 2: Package insert (9th edition) of "Type I Procollagen-N-propeptide Kit Elecsys Reagent (R) Total P1NP", Roche Diagnostics K.K., March 2021
Non-Patent Literature 3: Koivula, M. -K. , and 2 others, "Measurement of aminoterminal propeptide of type I procollagen (PINP) in serum", Clinical Biochemistry, August 2012, Vol. 45, No. 12, pp. 920-927
Non-Patent Literature 4: Koivula, M. -K. , and 7 others, "Difference between total and intact assays for N-terminal propeptide of type I procollagen reflects degradation of pN-collagen rather than denaturation of intact propeptide," Annals of Clinical Biochemistry, January 2010, Vol. 47, No. 1, p. 67-71

### [Summary of Invention]

### [Technical Problem]

Procollagen Intact PINP (hereinafter abbreviated as "Intact PINP") described in Non-Patent Literature 1 can detect only the trimer of the α-chain of type I collagen N-terminal propeptide. Intact PINP is a measurement kit based on radioimmunoassay method, and uses an antibody labeled with a radioisotope (¹²⁵I). Therefore, the measurement needs to be performed in a radiation controlled area and requires about 3-hour measurement time, which is cumbersome. In addition, since the half-life of the radioisotope (1251) is as short as 59.4 days, the shelf life of the kit is also as short as 6 weeks at 2 to 8 °C.

The Elecsys reagent total P1NP (hereinafter abbreviated as "total P1NP") described in Non-Patent Literature 2 can detect the trimer and monomer of the α-chain of type I collagen N-terminal propeptide. The total P1NP is a measurement kit based on electrochemiluminescence immunoassay (ECLIA), and is widely used because it is easy to handle with no need for a radiation control area and a measurement time of about 20 minutes. The kit also has a long shelf life of about 18 months at 2 to 8 °C.

There is a good correlation between the Intact PINP and the total P1NP, and it is assumed that there is no significant difference in performance between these products.

However, in patients with renal disease, clearance of the α-chain monomer of type I collagen N-terminal propeptide cannot be formed, so that the PINP value of samples from patients with renal disease is measured to be high when using total P1NP.

The present invention has been made in consideration of the above circumstances, and one of its objects is to provide an immunological detection method and an immunological detection kit that are easy to handle and can specifically (selectively) measure the trimer in biological samples containing the trimer and monomer of the α-chain of PINP.

### [Solution to Problem]

[1] An immunological detection method for trimeric type I collagen N-terminal propeptide in a biological sample, comprising utilizing:
   a first antibody that binds to a specific portion of a pro-al chain in the trimeric type I collagen N-terminal propeptide; and
   a second antibody that binds to a specific portion of a pro-α2 chain in the trimeric type I collagen N-terminal propeptide.
[2] The immunological detection method according to [1], wherein the first antibody is labeled with a labeling substance, and the method comprises:
   a step of contacting the biological sample and the first antibody to form a first complex;
   a step of contacting the first complex and the second antibody to form a second complex; and
   a step of detecting a signal derived from the labeling substance.
[3] The immunological detection method according to [1], wherein the second antibody is labeled with a labeling substance, and the method comprises:
   a step of contacting the biological sample and the second antibody to form a first complex;
   a step of contacting the first complex and the first antibody to form a second complex; and
   a step of detecting a signal derived from the labeling substance.
[4] The immunological detection method according to any one of [1] to [3], wherein a primary structure of the specific portion of the pro-α1 chain in the trimeric type I collagen N-terminal propeptide comprises a sequence represented by SEQ ID NO: 1 or SEQ ID NO: 2:
   SEQ ID NO: 1 QEEGQVEGQD
   SEQ ID NO: 2 PDGSESPTDQETT.
[5] The immunological detection method according to any one of [1] to [4], wherein a primary structure of the specific portion of the pro-α2 chain in the trimeric type I collagen N-terminal propeptide comprises a sequence represented by SEQ ID NO: 3:
   SEQ ID NO: 3 QSLQEETVRK.
[6] The immunological detection method according to any one of [1] to [5], wherein the first antibody reacts with a portion of a polypeptide having a sequence represented by SEQ ID NO: 4:
   SEQ ID NO: 4 JSLQEETVRK.
[7] The immunological detection method according to [2] or [3], wherein the biological sample is at least one selected from the group consisting of blood, plasma, and serum.
[8] The immunological detection method according to any one of [1] to [7], wherein at least one of the first antibody and the second antibody is a monoclonal antibody.
[9] The immunological detection method according to any one of [1] to [8], wherein the immunological detection method is any one selected from the group consisting of an electrochemiluminescence method, latex agglutination immunoturbidimetry, and enzyme-linked immunosorbent assay (ELISA).
[10] The immunological detection method according to any one of [1] to [9], which does not detect a monomer of type I collagen N-terminal propeptide derived from a renal disease patient.
[11] An immunological detection kit for trimeric type I collagen N-terminal propeptide in a biological sample, comprising:
   a first antibody that binds to a specific portion of a pro-α1 chain in the trimeric type I collagen N-terminal propeptide; and
   a second antibody that binds to a specific portion of a pro-α2 chain in the trimeric type I collagen N-terminal propeptide.
[12] The immunological detection kit according to [11], wherein the first antibody is labeled with a labeling substance;
   the biological sample and the first antibody are contacted to form a first complex;
   the first complex and the second antibody are contacted to form a second complex; and
   a signal derived from the labeling substance is detected.
[13] The immunological detection kit according to [11], wherein the second antibody is labeled with a labeling substance;
   the biological sample and the second antibody are contacted to form a first complex;
   the first complex and the first antibody are contacted to form a second complex; and
   a signal derived from the labeling substance is detected.
[14] The immunological detection kit according to any one of [11] to [13], wherein a primary structure of the specific portion of the pro-α1 chain in the trimeric type I collagen N-terminal propeptide comprises a sequence represented by SEQ ID NO: 1 or SEQ ID NO: 2:
   SEQ ID NO: 1 QEEGQVEGQD
   SEQ ID NO: 2 PDGSESPTDQETT.
[15] The immunological detection kit according to any one of [11] to [14], wherein a primary structure of the specific portion of the pro-α2 chain in the trimeric type I collagen N-terminal propeptide comprises a sequence represented by SEQ ID NO: 3:
   SEQ ID NO: 3 QSLQEETVRK.
[16] The immunological detection kit according to any one of [11] to [15], wherein the first antibody reacts with a portion of a polypeptide having a sequence represented by SEQ ID NO: 4:
   SEQ ID NO: 4 JSLQEETVRK.
[17] The immunological detection kit according to [12] or [13], wherein the biological sample is at least one selected from the group consisting of blood, plasma, and serum.
[18] The immunological detection kit according to any one of [11] to [17], wherein at least one of the first antibody and the second antibody is a monoclonal antibody.
[19] The immunological detection kit according to any one of claims [11] to [18], which utilizes any one immunological detection method selected from the group consisting of an electrochemiluminescence method, latex agglutination immunoturbidimetry, and enzyme-linked immunosorbent assay (ELISA).
[20] The immunological detection kit according to any one of [11] to [19], which does not detect a monomer of type I collagen N-terminal propeptide derived from a renal disease patient.

### [Advantageous Effects of Invention]

The present invention can provide an immunological detection method and an immunological detection kit that are easy to handle and can specifically (selectively) measure the trimer in biological samples containing the trimer and monomer of the α-chain of PINP.

### [Brief Description of Drawings]

FIG. 1 is a schematic diagram illustrating the specific portions of pro-al chain and pro-α2 chain.
FIG. 2 is a schematic diagram illustrating the amino acid sequence with which antibody S24201 reacts.
FIG. 3 is a graph showing the measurement results for osteoporosis patients and healthy subjects by ELISA.
FIG. 4 is a graph showing the measurement results for osteoporosis patients and healthy subjects by ELISA.
FIG. 5 is a graph showing the measurement results for osteoporosis patients and healthy subjects using commercially available reagents.
FIG. 6 is a graph showing the measurement results for osteoporosis patients and renal disease patients by electrochemiluminescence.
FIG. 7 is a graph showing the measurement results for osteoporosis patients and renal disease patients by electrochemiluminescence.
FIG. 8 is a graph showing the measurement results for samples from renal disease patients by ELISA.
FIG. 9 is a graph showing the measurement results for samples from renal disease patients by ELISA.
FIG. 10 is a graph showing the measurement results for samples from renal disease patients using commercially available reagents.

### [Description of Embodiments]

Hereinbelow, embodiments of the present invention are described, but the present invention is not limited to such embodiments, and various modifications can be made as long as such modifications do not deviate from the substance of the present invention.

In the present invention, the one-letter abbreviations for amino acids follow those described in IUPAC-IUB Joint Commission on Biochemical Nomenclature (JCBN), Nomenclature and Symbolism for Amino Acids and Peptides Recommendations 1983, European Journal of Biochemistry, 1984, Vol. 138, pp. 9-37. However, in the present invention, the one-letter abbreviation for "pyroglutamic acid" is "J". The amino acid sequence is described in the order of from N-terminus to C-terminus.

In the present invention, the terms "react," "recognize," and "bind" are used synonymously in relation to the reactivity of an antibody with an antigen. When an antibody disclosed herein is described as not reactive with respect to a certain compound, it means that the antibody does not substantially react with the certain compound. For example, in the Examples described below, the antibody is described as not reactive when the measured value is below the detection limit.

In the present invention, the terms "detection" and "measurement" are used in a sense encompassing the verification of the presence and/or quantification of trimeric type I collagen N-terminal propeptide.

### [Immunological detection method]

The immunological detection method of the present invention is an immunological detection method for trimeric type I collagen N-terminal propeptide in a biological sample, and uses a first antibody that binds to a specific portion of a pro-α1 chain in the trimeric type I collagen N-terminal propeptide and a second antibody that binds to a specific portion of a pro-α2 chain in the trimeric type I collagen N-terminal propeptide.

### <Biological sample>

The biological sample that can be used in the immunological detection method of the present invention is not particularly limited as long as it is a sample derived from a living body, but is preferably a liquid sample such as a body fluid, more preferably at least one selected from the group consisting of blood, plasma, and serum, and even more preferably any one selected from the group consisting of blood, plasma, and serum. The living body is preferably a human or a mammal (e.g., a mouse, a guinea pig, a rat, a monkey, a dog, a cat, a hamster, a horse, a cow, and a pig), and more preferably a human. The biological sample may be one collected or prepared during the implementation of the present invention, or may be one collected or prepared in advance and stored. The biological sample may usually contain both the trimer and monomer of type I collagen N-terminal propeptide, or may contain only one of the trimer and the monomer, or may contain neither the trimer nor the monomer.

The biological sample may be derived from a subject with impaired renal function. Examples of subjects with impaired renal function include patients with chronic renal disease. Renal function can be evaluated, for example, by eGFR value (estimated glomerular filtration rate, unit: mL/min/1.73m²).

| | |
|---|---|
| eGFR ≥ 90 | Normal |
| eGFR = 60 to 89 | Mild renal impairment |
| eGFR = 30 to 59 | Moderate renal impairment |
| eGFR = 15 to 29 | Severe renal impairment |
| eGFR < 15 | End-stage renal disease |

### <Trimeric type I collagen N-terminal propeptide>

Trimeric type I collagen N-terminal propeptide is a polypeptide with a molecular weight of approximately 35 kDa, which is cleaved from the N-terminus during the formation of type I collagen from its precursor, i.e., type I procollagen. This polypeptide is a heterotrimer consisting of two α1 chains (pro-α1 chains) with a molecular weight of approximately 15 kDa and one α2 chain (pro-α2 chain) with a molecular weight of approximately 5 kDa.

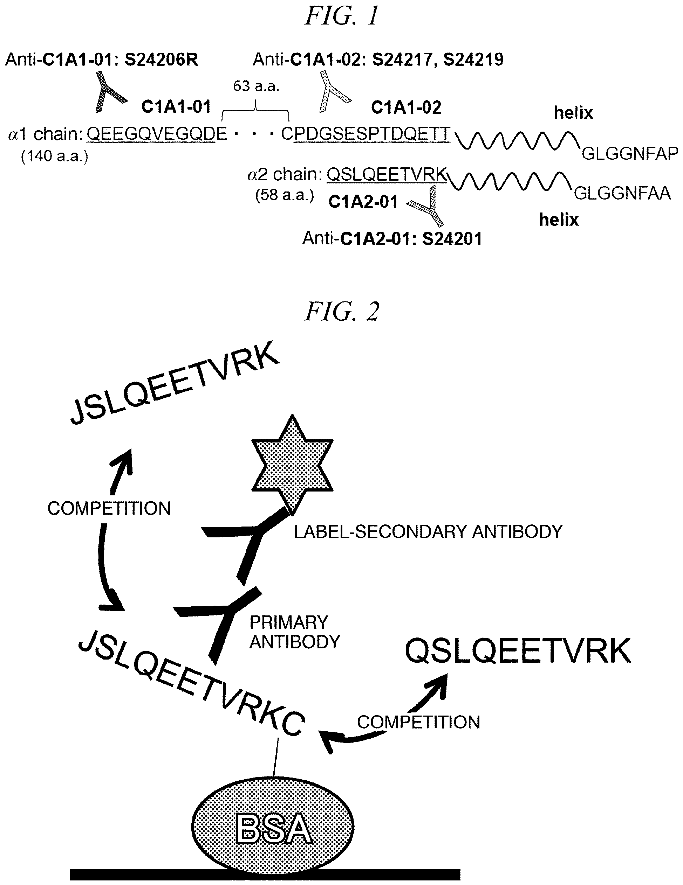

The specific portion of the pro-α1 chain is a portion having a specific primary structure (amino acid sequence) to which the first antibody binds. The primary structure (amino acid sequence) of the specific portion of the pro-α1 chain is, for example, QEEGQVEGQD (SEQ ID NO: 1) or PDGSESPTDQETT (SEQ ID NO: 2) shown in FIG. 1.

The specific portion of the pro-α2 chain is a portion having a specific primary structure (amino acid sequence) to which the second antibody binds. The primary structure (amino acid sequence) of the specific portion of the pro-α2 chain is, for example, QSLQEETVRK (SEQ ID NO: 3) shown in FIG. 1 or JSLQEETVRK (SEQ ID NO: 4) in which the N-terminal Q (glutamine residue) of the amino acid sequence of SEQ ID NO: 3 is replaced with J (pyroglutamate residue). It is known that in living bodies, deamidation of the N-terminal glutamine side chain and condensation with an amino group occur to generate a cyclic pyroglutamyl residue, and the N-terminus of the subunit of type I collagen also contains a pyroglutamate residue ("Modified Peptides in Foods - Structure and Function of Pyroglutamyl Peptides," Kenji Sato, Tamami Kiyono, Foods & Food Ingredients Journal, 2017, Vol. 222, No. 3, pp. 216-222).

### <First antibody>

The first antibody is not particularly limited as long as it binds to a specific portion of the pro-al chain, but is preferably an antibody that binds to the primary structure (amino acid sequence) of QEEGQVEGQDC (SEQ ID NO: 5) or PDGSESPTDQETTC (SEQ ID NO: 6) shown in FIG. 1, such as an anti-C1A1-01 antibody and an anti-C1A1-02 antibody.

The first antibody is preferably a monoclonal antibody. A monoclonal antibody can be produced, for example, by fusing a B lymphocyte with an immortalized cultured cell to form a hybridoma. Monoclonal antibodies are preferable because they are specific and are expected to produce significantly lower background signals than polyclonal antibodies.

The anti-C1A1-01 antibody is an antibody prepared by the method of the Examples described below using an immunogen obtained by binding ovalbumin, a carrier protein, to a peptide having a primary structure represented by SEQ ID NO: 5: QEEGQVEGQDC. The hybridoma producing the antibody S24206R shown in FIG. 1 has been deposited at the National Institute of Technology and Evaluation, Patent Microorganism Depositary (NPMD) (Room 122, 2-5-8 Kazusakamatari, Kisarazu, Chiba Prefecture, Japan) under the name NITE BP-03606 (deposit date: February 15, 2022).

The anti-C1A1-02 is an antibody prepared by the method of the Examples described below using an immunogen obtained by binding ovalbumin, a carrier protein, to a peptide having a primary structure represented by SEQ ID NO: 6: PDGSESPTDQETTC. The hybridoma producing the antibody S24217 shown in FIG. 1 has been deposited at the NPMD as NITE BP-03607, and the hybridoma producing the antibody S24219 has been deposited at the NPMD as NITE BP-03608 (deposit date: February 15, 2022).

### <Second antibody>

The second antibody is not particularly limited as long as it binds to a specific portion of the pro-α2 chain, but is preferably an antibody that binds to the primary structure (amino acid sequence) of QSLQEETVRK (SEQ ID NO: 3) shown in FIG. 1, such as an anti-C1A2-01 antibody shown in FIG. 1.

The second antibody is preferably a monoclonal antibody. A monoclonal antibody can be produced, for example, by fusing a B lymphocyte with an immortalized cultured cell to form a hybridoma. Monoclonal antibodies are preferable because they are specific and are expected to produce significantly lower background signals than polyclonal antibodies.

The anti-C1A2-01 antibody is an antibody prepared by the method described in the Examples below using an immunogen obtained by binding ovalbumin, a carrier protein, to a peptide having a primary structure represented by SEQ ID NO: 7: JSLQEETVRKC. The hybridoma producing the antibody S24201 shown in FIG. 1 has been deposited at the NPMD as NITE BP-03605.

As shown in FIG. 2, the antibody S24201 reacts with both of the polypeptides having the primary structures (amino acid sequences) QSLQEETVRK (SEQ ID NO: 3) and JSLQEETVRK (SEQ ID NO: 4).

### <Detection method>

The detection method is not particularly limited as long as it is an immunological method, but is preferably any one selected from the group consisting of an electrochemiluminescence method, latex agglutination immunoturbidimetry, and enzyme-linked immunosorbent assay (ELISA).

For detection, it is preferable that at least one of the first antibody and the second antibody is labeled with a labeling substance. Examples of the labeling substance include enzymes, fluorescent substances, chemiluminescent substances, biotin, avidin, gold colloid particles, and colored latex.

In the detection method of the present invention, the following embodiments (1) or (2) are preferred.
(1) The first antibody is labeled with a labeling substance, the biological sample is contacted with the first antibody to form a first complex, the first complex is contacted with the second antibody to form a second complex, and a signal derived from the labeling substance is detected.
(2) The second antibody is labeled with a labeling substance, the biological sample is contacted with the second antibody to form a first complex, the first complex is contacted with the first antibody to form a second complex, and a signal derived from the labeling substance is detected.

The method for detecting a signal derived from a labeling substance can be appropriately selected depending on the respective labeling substances.

### [Immunological detection kit]

The immunological detection kit of the present invention is an immunological detection kit for trimeric type I collagen N-terminal propeptide in a biological sample, and includes a first antibody that binds to a specific portion of a pro-al chain in the trimeric type I collagen N-terminal propeptide, and a second antibody that binds to a specific portion of a pro-α2 chain in the trimeric type I collagen N-terminal propeptide.

The biological sample, trimeric type I collagen N-terminal propeptide, the first antibody, and the second antibody are as described for the immunological detection method of the present invention.

The immunological detection kit of the present invention may further include a protocol, a detection device, an analysis device, etc.

The immunological detection kit of the present invention is preferable for carrying out the above-mentioned immunological detection method of the present invention.

### EXAMPLES

Hereinbelow, the present invention will be described with reference to Examples which, however, should not be construed as limiting the present invention.

### 1. Preparation of immunogen

The following peptides were bound to the carrier protein ovalbumin (OVA) to prepare immunogens.
C1A1-01: QEEGQVEGQDC (SEQ ID NO: 5)
Uniprot ID: P02452, type I procollagen α1, Gln23-Asp32+Cys
C1A1-02: PDGSESPTDQETTC (SEQ ID NO: 6)
Uniprot ID: P02452, type I procollagen α1, Pro96-Thr108+Cys
C1A2-01: JSLQEETVRKC (SEQ ID NO: 7)
Uniprot ID: P08123, type I procollagen α2, Q23-Asp32+Cys
   Q23 was converted to J. In SEQ ID NO: 7, J represents pyroglutamic acid.

(1) 0.2 mL of purified water was added to Imject Maleimide Activated OVA (Thermo Fischer) to obtain a 10 mg/mL solution.
(2) 2 mg of peptides (C1A1-01, C1A1-02) were each dissolved in 0.2 mL of PBS (phosphate buffered saline) to obtain peptide solutions with a concentration of 10 mg/mL. In addition, 2 mg of peptide (C1A2-01) was dissolved in a small amount of DMSO (dimethyl sulfoxide), followed by addition of PBS to obtain a peptide solution with a concentration of 3.3 mg/mL.
(3) The three peptide solutions obtained in (1) and (2) were individually stirred at room temperature for 2 hours.
(4) The solutions were dialyzed twice against 500 mL of PBS to obtain C1A1-01-OVA, C1A2-01-OVA, and C1A1-02-OVA solutions.

### 2. Antibody acquisition

C1A1-01-OVA, C1A2-01-OVA, and C1A1-02-OVA were used as immunogens, and were mixed at 1:1 with Freund's Complete Adjuvant (Difco Laboratories) for the first immunization and Freund's Incomplete Adjuvant (Difco Laboratories) for the second and subsequent immunizations. 6-week-old (female) Balb/c mice (CLEA Japan, Inc.) or F344/Jc1 rats (CLEA Japan, Inc.) were subcutaneously immunized every 2 weeks with 40 µg of the immunogen for the first immunization and 20 µg for the second and subsequent immunizations. The immunization was continued for 8 weeks. Antigen-immobilized ELISA (enzyme-linked immunosorbent assay) was performed according to the following procedure to determine the antibody titer in the blood.

(1) 50 µL/well each of C1A1-01-OVA, C1A2-01-OVA and C1A1-02-OVA solutions, each adjusted to 1 µg/mL with PBS, were dispensed into a 96-well plate and allowed to stand at room temperature for 2 hours.
(2) After washing three times with 400 µL/well of PBST (phosphate-buffered saline with Tween^{®}), 1% BSA/PBST (blocking solution) was dispensed and the resulting was allowed to stand at room temperature for 1 hour.
(3) After removing the blocking solution, 50 µL/well each of antiserum solutions from respective immunized animals, diluted 500 to 121,500-fold with 1% BSA/PBST, were dispensed, and the resulting was allowed to stand at room temperature for 1 hour.
(4) The antiserum solutions were removed, and the plate was washed three times with 400 µL/well of PBST. Then, 50 µL/well of Gt anti-Mouse IgG (H+L) PAb-HRP (SouthernBiotech) diluted 95,000-fold with 1% BSA/PBST or 50 µL/well of Gt anti-Rat IgG (H+L) PAb-HRP (SouthernBiotech) diluted 8,500-fold with 1% BSA/PBST was dispensed, and the resulting was allowed to stand at room temperature for 1 hour.
(5) The antibody solution was removed, and the plate was washed three times with 400 µL/well of PBST. Then, 50 µL/well of OPD (o-phenylenediamine dihydrochloride) coloring solution was dispensed, and the resulting was allowed to stand at room temperature for 10 minutes.
(6) After dispensing 50 µL/well of stop solution to stop the reaction, the absorbance at a wavelength of 492 nm was measured using a plate reader.

Individuals showing a sufficient increase in antibody titer were intraperitoneally immunized with 50 µg of each immunogen diluted in PBS 1 to 3 days before dissection. Then, spleen cells, iliac lymph node cells, and inguinal lymph node cells were collected and fused with myeloma cells SP2/0 by electrofusion method. The fused cells were cultured in a 96-well plate, and the culture supernatant was collected 7 or 8 days after the fusion. The above-mentioned antigen-immobilized ELISA was performed while replacing the antiserum solution with the culture supernatant, and cell lines producing antibodies that reacted with the immunogens were selected to obtain the following 4 antibody clones.

**[Table 1]**

| Target | Immunogen | Immunogen peptide sequence | Acquired antibody | Hybridoma deposit No. |
|---|---|---|---|---|
| α1 chain | C1A1-01 | QEEGQVEGQDC | S24206R | NITE BP-03606 |
| | C1A1-02 | PDGSESPTDQETTC | S24217 | NITE BP-03607 |
| | | | S24219 | NITE BP-03608 |
| α2 chain | C1A2-01 | JSLQEETVRKC | S24201 | NITE BP-03605 |

### 3. Competitive ELISA

0.2 mL of purified water was added to Imject Maleimide Activated BSA (Thermo Fischer) to obtain a 10 mg/mL solution. A peptide solution prepared by dissolving 2 mg of C1A2-01 peptide in a small amount of DMSO, followed by addition of PBS to adjust the peptide concentration to 3.3 mg/mL was added, and the resulting was stirred at room temperature for 2 hours. The resulting was dialyzed against PBS to obtain C1A2-01-BSA.

50 µL/well of C1A1-02-BSA solution adjusted to 25 ng/mL with PBS was dispensed into a 96-well plate and allowed to stand at room temperature for 2 hours. After washing three times with 400 µL/well of PBST, 1% BSA/PBST (blocking solution) was dispensed and the resulting was allowed to stand at room temperature for 1 hour. After removing the blocking solution, 25 µL/well of peptide C1A2-02 (JSLQEETVRK: SEQ ID NO: 4) or peptide C1A2-03 (QSLQEETVRK: SEQ ID NO: 3) solution dissolved in 1% BSA/PBST at a concentration of 0.32 to 5000 ng/mL, and 25 µL/well of S24201 antibody solution diluted in 1% BSA/PBST to a concentration of 125 ng/mL were dispensed, and the resulting was allowed to stand at room temperature for 1 hour. After removing the peptide solution and antibody solution and washing three times with 400 µL/well of PBST, 50 µL/well of Gt anti-Mouse IgG (H+L) PAb-HRP (SouthernBiotech) diluted 8000-fold with 1% BSA/PBST was dispensed and the resulting was allowed to stand at room temperature for 1 hour. After removing the antibody solution and washing three times with 400 µL/well of PBST, 50 µL/well of OPD coloring solution was dispensed and the resulting was allowed to stand at room temperature for 10 minutes. After dispensing 50 µL/well of stop solution to stop the reaction, the absorbance at a wavelength of 492 nm was measured using a plate reader.

### 4. PINP value measurement using commercially available reagents

The samples were measured using Procollagen Intact PINP (Fujirebio Inc.) and Elecsys Reagent Total P1NP (Roche Diagnostics K.K.) by an outside contractor.

### 5. Sandwich ELISA

(1) A solid-phase antibody prepared at 5 µg/mL in PBS was dispensed onto an ELISA plate (50 µL/well) and allowed to stand at room temperature for 2 hours.
(2) After washing three times with PBST (400 µL/well), 1% BSA/PBST (blocking solution) was dispensed (100 µL/well) and the resulting was allowed to stand at room temperature for 1 hour.
(3) After removing the blocking solution, a sample diluted 10-fold with 1% BSA/PBST was dispensed (50 µL/well) and the resulting was allowed to stand at room temperature for 1 hour.
(4) After washing three times (400 µL/well), a solution of biotinylated antibody, which had been biotinylated as per standard method using Biotin Labeling Kit-NH2 (Dojindo Laboratories), and adjusted to 0.2 µg/mL in 1% BSA/PBST, was dispensed (50 µL/well), and the resulting was allowed to stand at room temperature for 1 hour.
(4) After washing three times (400 pL/well), HRP-Streptavidin adjusted to 0.2 µg/mL in PBS was dispensed (50 µL/well) and the resulting was allowed to stand at room temperature for 30 minutes.
(5) After washing three times, an OPD coloring solution was dispensed (50 µL/well) and the resulting was allowed to stand at room temperature for 10 minutes.
(6) A stop solution was dispensed (50 µL/well), and the absorbance was measured using a plate reader (wavelength: 492 nm).

### 6. Electrochemiluminescence Method

### (1) Preparation of S24217 antibody-immobilized magnetic particle suspension

10 mg of magnetic particles (manufactured by Sekisui Medical Co., Ltd.) with a diameter of 3 µm were washed with PBS to remove the solution. A S24217 antibody solution diluted with PBS to give an absorbance of 0.5 at 280 nm was added to the magnetic particles and the resulting was stirred at room temperature for 20 hours. The antibody solution was removed, and the magnetic particles were washed three times with 1 mL of blocking solution (0.1% BSA, 50 mM HEPES, 150 mM NaCl, 0.01% Tween-20, 0.05% ProClin 300, pH 7.2). 500 µL of the blocking solution was added, and after stirring at room temperature for 16 hours, the resulting was diluted with the blocking solution to 0.5 mg/mL to obtain an S24217 antibody-immobilized magnetic particle suspension.

### (2) Preparation of ruthenium (Ru) complex-labeled S24201 antibody solution

S24201 antibody was prepared in PBS to a concentration of 2.0 mg/mL, followed by adding, with stirring, 20 equivalents of bis(2,2'-bipyridine) [4'-methyl-4-(4-NHS-4-oxobutyl)-2,2'bipyridine] ruthenium (II) bis(hexafluorophosphate) (hereinafter referred to as Ru complex NHS, manufactured by Tokyo Chemical Industry Co., Ltd.) per mole of the antibody, which had been dissolved in a small amount of DMSO, and the resulting was stirred for 30 minutes at room temperature in the dark. 2M glycine was added while stirring so that the molar ratio of glycine to Ru complex NHS was 2:1, and the resulting was stirred for 20 minutes in the dark. Using Sephadex G-25 equilibrated with PBS, Ru complexes not bound to the antibody were removed from the resulting solution, and the solution was diluted to 1 µg/mL with a blocking solution to obtain a Ru complex-labeled S24201 antibody solution.

### (3) Electrochemiluminescence measurement

A fully automated electrochemiluminescence immunoassay device PicoLumi III (manufactured by Sekisui Medical Co., Ltd.) was used for the measurement. 100 µL of blocking solution containing mouse IgG and 5 µL of the measurement sample were added to a reaction tube. 25 µL of S24217 antibody-immobilized magnetic particle suspension was added and reacted for 5 minutes at 28 °C. After separation of BF using the BF separation solution specified for the device, 100 µL of Ru complex-labeled S24201 antibody solution was added and the resulting was reacted for 3 minutes. After the BF separation, the magnetic particles were suspended in the substrate (tripropylamine) solution specified for the device, and electricity was applied at a predetermined position on the device.

### [Example 1]

### <Reactivity of the obtained anti-α2 chain antibody with peptides>

The reactivity of the S24201 antibody with the immunogens, i.e., C1A2-02 peptide (JSLQEETVRK) and C1A2-03 peptide (QSLQEETVRK), which is generally known to be part of the P1NP α2 chain sequence, was examined by competitive ELISA. As shown in Table 2, for both peptides, the ELISA absorbance decreased as the competitive peptide concentration increased. This means that the S24201 antibody reacted with both the C1A2-02 peptide and the C1A2-03 peptide.

**[Table 2]**

| | Absorbance at wavelength 492 nm | |
|---|---|---|
| Competitive peptide concentration (ng/ml) | C1A2-02 peptide (JSLQEETVRK) | C1A2-03 peptide (QSLQEETVRK) |
| 5000 | 0.074 | 0.313 |
| 1000 | 0.142 | 0.521 |
| 200 | 0.306 | 0.652 |
| 40 | 0.541 | 0.693 |
| 8 | 0.668 | 0.681 |
| 1.6 | 0.735 | 0.7 |
| 0.32 | 0.712 | 0.706 |
| 0 | 0.752 | 0.702 |

### [Example 2]

### <Measurement of samples from osteoporosis patients and healthy subjects by ELISA>

16 Samples from healthy subjects and 22 samples from osteoporosis patients without renal disease were used.

Sandwich ELISA was performed using the antibody combinations shown in Table 3. The same samples were also measured with Procollagen Intact PINP and Elecsys Reagent Total P1NP.

**[Table 3]**

| | Solid-phase antibody | Biotinylated antibody |
|---|---|---|
| Antibody combination 1 | S24201 | S24206R |
| Antibody combination 2 | S24206R | S24201 |

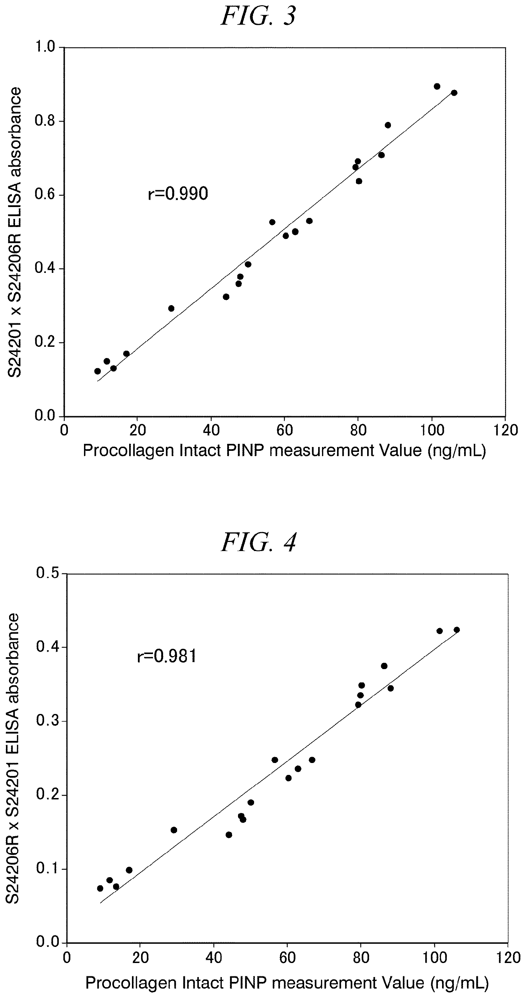

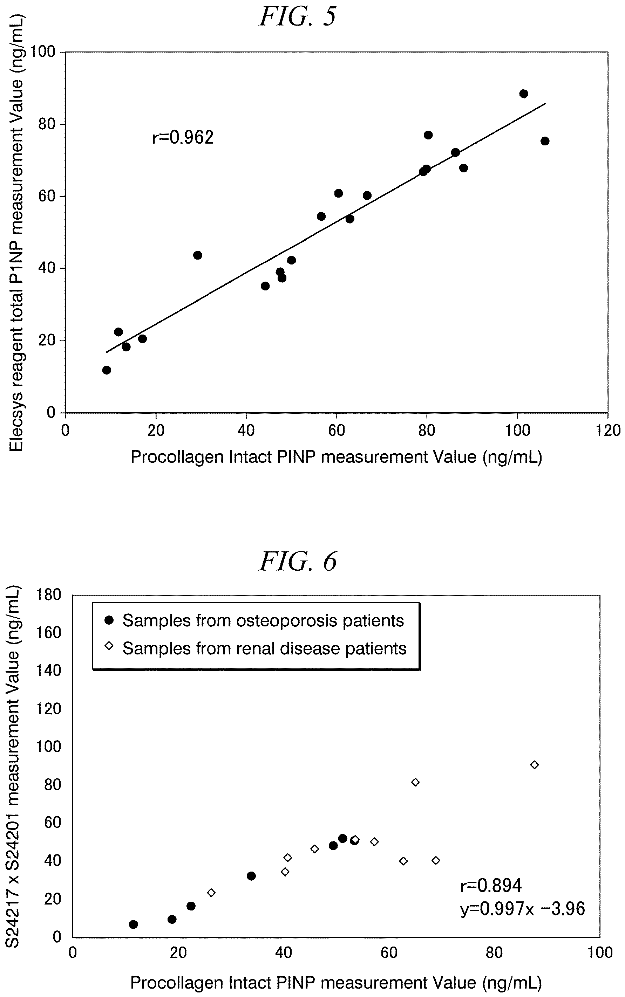

The results are shown in FIGs. 3 to 5. The Pearson's product moment correlation coefficient between the ELISA absorbance values using the antibody combination 1 and the Procollagen Intact PINP measurement values was: r = 0.990 (FIG. 3). The Pearson's product moment correlation coefficient between the ELISA absorbance values using the antibody combination 2 and the Procollagen Intact PINP measurement values was: r = 0.981 (FIG. 4). The Pearson's product moment correlation coefficient between the Elecsys reagent total P1NP measurement values and the Procollagen Intact PINP measurement values was: r = 0.962 (FIG. 5). Therefore, there was a good correlation between the absorbance values by the sandwich ELISA for each antibody combination and the measurement values by the Procollagen Intact PINP.

### [Example 3]

### <Measurement of samples from patients with osteoporosis and renal disease using electrochemiluminescence method>

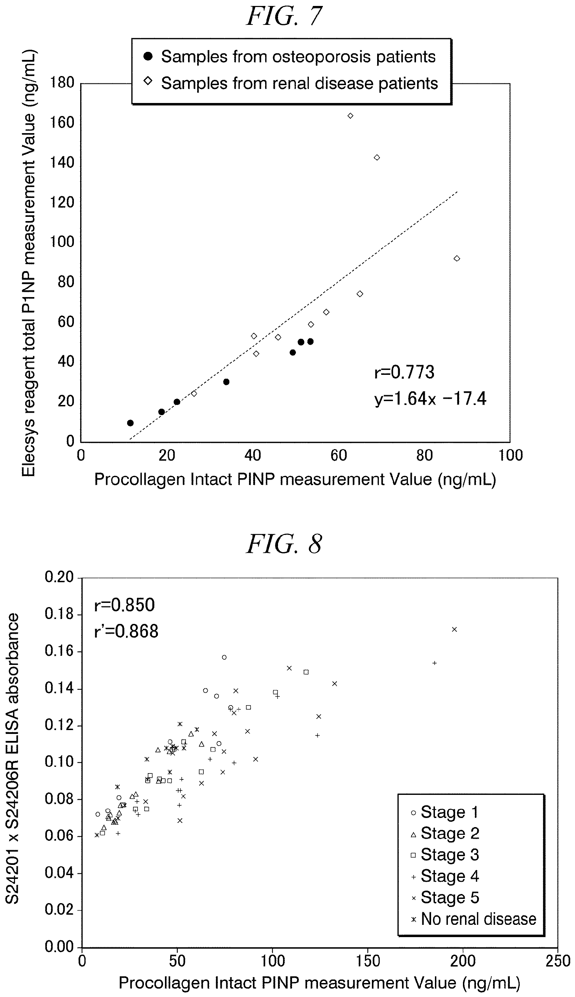

10 Samples from patients with renal disease and 7 samples from patients with osteoporosis were used. Serial dilutions of serum from patients with cancer bone metastasis with high P1NP levels, valued the Elecsys reagent total P1NP, were used as standard, and P1NP measurements were calculated using electrochemiluminescence method using S24217 as a solid-phase antibody and S24201 as a Ru complex-labeled antibody. The results are shown in FIG. 6 and FIG. 7. The correlation between the values measured by the electrochemiluminescence method of the present invention and the values measured by Procollagen Intact PINP was: r = 0.894, y = 0.997x - 3.96 in terms of Pearson's product moment correlation coefficient. On the other hand, the correlation between the valuesmeasured by the Elecsys reagent total P1NP and the valuesmeasured by Procollagen Intact PINP was r = 0.773, y = 1.64x - 17.4 in terms of Pearson's product moment correlation coefficient. Therefore, even when a group of samples including samples from patients with renal disease was measured, there was a good correlation between the values measured by the electrochemiluminescence method using S24217 as the solid-phase antibody and S24201 as the Ru complex-labeled antibody and the values measured by Procollagen Intact PINP.

### [Example 4]

### <Measurement of samples from patients with renal disease using ELISA>

3 Samples from healthy individuals, 10 samples from osteoporosis patients without renal disease, and 75 samples from patients with renal disease were used.

Sandwich ELISA was performed using the antibody combinations shown in Table 3. The same samples were also measured with Procollagen Intact PINP and Elecsys Reagent Total P1NP.

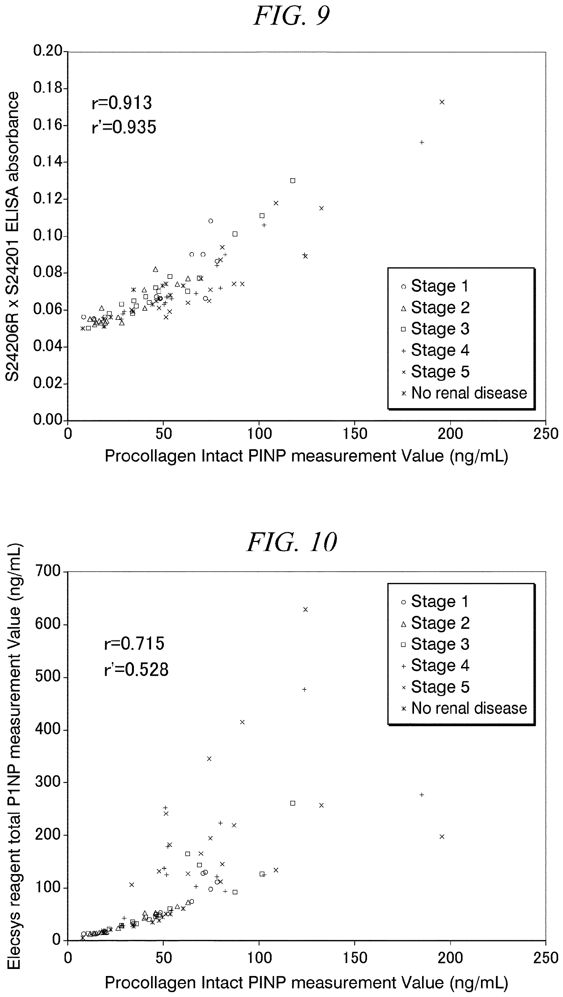

The results are shown in FIG. 8 and FIG. 9. The Pearson's product moment correlation coefficient between the ELISA absorbance values using the antibody combination 1 and the Procollagen Intact PINP measurement values was: r = 0.850 for all samples, and r' = 0.868 for samples from patients at stages 4 and 5. The Pearson's product moment correlation coefficient between the ELISA absorbance values using the antibody combination 2 and the Procollagen Intact PINP measurement values was: r = 0.913 for all samples, and r' = 0.935 for samples from patients at stages 4 and 5. For all antibody combinations, there was a good correlation between the absorbance values by the sandwich ELISA of the present invention and the measurement values by the Procollagen Intact PINP.

On the other hand, as shown in FIG. 10, the Pearson's product moment correlation coefficient between the measurement values by the Elecsys reagent total P1NP and the measurement valuesby the Procollagen Intact PINP was: r = 0.715 for all samples, and r' = 0.528 for samples from patients at stages 4 and 5. The correlation between the measurement values by the Elecsys reagent total P1NP and the measurement values by the Procollagen Intact PINP was particularly poor in samples from patients with severe renal disease.

The relationship between renal disease (chronic renal disease) stage and eGFR (unit: mL/min/1.73 m²) is as follows:
Stage 1: eGFR ≥ 90
Stage 2: eGFR = 60 to 89
Stage 3a: eGFR = 45 to 59
Stage 3b: eGFR = 30 to 44
Stage 4: eGFR = 15 to 29
Stage 5: eGFR < 15

### [Sequence listing]

**[Table 4]**

| SEQ ID NO. | Amino acid sequence (N terminus to C terminus) |
|---|---|
| 1 | QEEGQVEGQD |
| 2 | PDGSESPTDQETT |
| 3 | QSLQEETVRK |
| 4 | JSLQEETVRK |
| 5 | QEEGQVEGQDC |
| 6 | PDGSESPTDQETTC |
| 7 | JSLQEETVRKC |

*In Table 4, J represents pyroglutamic acid.

### Accession number

NPMD NITE BP-03606
NPMD NITE BP-03607
NPMD NITE BP-03608
NPMD NITE BP-03605

"NPMD" is an acronym for the National Institute of Technology and Evaluation, Patent Microorganism Depositary (Room 122, 2-5-8 Kazusakamatari, Kisarazu, Chiba Prefecture, Japan).

| | | |
|---|---|---|
| 0-1 | Form PCT/RO/134 | |
| | Indications Relating to Deposited Microorganism (s) or Other Biological | |
| | Material (PCT Rule 13bis) | JPO-PAS |
| | Prepared Using | |
| 0-1-1 | | i470 |
| 0-2 | International Application No. | |
| 0-3 | Applicant's or agent's file reference | PC-36340 |

| | | |
|---|---|---|
| 1 | The indications made below relate to the deposited microorganism(s) or other biological material referred to in the description on: | |
| 1-1 | paragraph | 65 |
| 1-3 | Identification of deposit | |
| 1-3-1 | Name of depositary Institution | NPMD National Institute of Technology and Evaluation, Patent Microorganism Depositary (NPMD) |
| 1-3-2 | Address of depositary Institution | Room 122, 2-5-8 Kazusakamatari, Kisarazu, Chiba Prefecture, 292-0818 Japan |
| 1-3-3 | Date of deposit | 15 February 2022 (15.02.2022) |
| 1-3-4 | Accession Number | NPMD NITE BP-03605 |
| 1-5 | Designated States for Which Indications are Made | All designations |
| 2 | The indications made below relate to the deposited microorganism(s) or other biological material referred to in the description on: | |
| 2-1 | paragraph | 65 |
| 2-3 | Identification of deposit | |
| 2-3-1 | Name of depositary Institution | NPMD National Institute of Technology and Evaluation, Patent Microorganism Depositary (NPMD) |
| 2-3-2 | Address of depositary Institution | Room 122, 2-5-8 Kazusakamatari, Kisarazu, Chiba Prefecture, 292-0818 Japan |
| 2-3-3 | Date of deposit | 15 February 2022 (15.02.2022) |
| 2-3-4 | Accession Number | NPMD NITE BP-03606 |
| 2-5 | Designated States for Which Indications are Made | All designations |
| 3 | The indications made below relate to the deposited microorganism(s) or other biological material referred to in the description on: | |
| 3-1 | paragraph | 65 |
| 3-3 | Identification of deposit | |
| 3-3-1 | Name of depositary Institution | NPMD National Institute of Technology and Evaluation, Patent Microorganism Depositary (NPMD) |
| 3-3-2 | Address of depositary Institution | Room 122, 2-5-8 Kazusakamatari, Kisarazu, Chiba Prefecture, 292-0818 Japan |
| 3-3-3 | Date of deposit | 15 February 2022 (15.02.2022) |
| 3-3-4 | Accession Number | NPMD NITE BP-03607 |
| 3-5 | Designated States for Which Indications are Made | All designations |
| 4 | The indications made below relate to the deposited microorganism(s) or other biological material referred to in the description on: | |
| 4-1 | paragraph | 65 |
| 4-3 | Identification of deposit | |
| 4-3-1 | Name of depositary Institution | NPMD National Institute of Technology and Evaluation, Patent Microorganism Depositary (NPMD) |
| 4-3-2 | Address of depositary Institution | Room 122, 2-5-8 Kazusakamatari, Kisarazu, Chiba Prefecture, 292-0818 Japan |
| 4-3-3 | Date of deposit | 15 February 2022 (15.02.2022) |
| 4-3-4 | Accession Number | NPMD NITE BP-03608 |
| 4-5 | Designated States for Which Indications are Made | All designations |

### FOR RECEIVING OFFICE USE ONLY

| | | |
|---|---|---|
| 0-4 | This form was received with the international application: (yes or no) | YES |
| 0-4-1 | Authorized officer | Nagai Tsuneo |

### FOR INTERNATIONAL BUREAU USE ONLY

| | | |
|---|---|---|
| 0-5 | This form was received by the international Bureau on: | |
| 0-5-1 | Authorized officer | |

## Claims

1. An immunological detection method for trimeric type I collagen N-terminal propeptide in a biological sample, comprising utilizing:
a first antibody that binds to a specific portion of a pro-al chain in the trimeric type I collagen N-terminal propeptide; and
a second antibody that binds to a specific portion of a pro-α2 chain in the trimeric type I collagen N-terminal propeptide.

2. The immunological detection method according to claim 1, wherein the first antibody is labeled with a labeling substance, and the method comprises:
a step of contacting the biological sample and the first antibody to form a first complex;
a step of contacting the first complex and the second antibody to form a second complex; and
a step of detecting a signal derived from the labeling substance.

3. The immunological detection method according to claim 1, wherein the second antibody is labeled with a labeling substance, and the method comprises:
a step of contacting the biological sample and the second antibody to form a first complex;
a step of contacting the first complex and the first antibody to form a second complex; and
a step of detecting a signal derived from the labeling substance.

4. The immunological detection method according to any one of claims 1 to 3, wherein a primary structure of the specific portion of the pro-α1 chain in the trimeric type I collagen N-terminal propeptide comprises a sequence represented by SEQ ID NO: 1 or SEQ ID NO: 2:
| | |
|---|---|
| SEQ ID NO: 1 | QEEGQVEGQD |
| SEQ ID NO: 2 | PDGSESPTDQETT. |

5. The immunological detection method according to any one of claims 1 to 3, wherein a primary structure of the specific portion of the pro-α2 chain in the trimeric type I collagen N-terminal propeptide comprises a sequence represented by SEQ ID NO: 3:
| | |
|---|---|
| SEQ ID NO: 3 | QSLQEETVRK. |

6. The immunological detection method according to any one of claims 1 to 3, wherein the first antibody reacts with a portion of a polypeptide having a sequence represented by SEQ ID NO: 4:
| | |
|---|---|
| SEQ ID NO: 4 | JSLQEETVRK. |

7. The immunological detection method according to claim 2 or 3, wherein the biological sample is at least one selected from the group consisting of blood, plasma, and serum.

8. The immunological detection method according to any one of claims 1 to 3, wherein at least one of the first antibody and the second antibody is a monoclonal antibody.

9. The immunological detection method according to any one of claims 1 to 3, wherein the immunological detection method is any one selected from the group consisting of an electrochemiluminescence method, latex agglutination immunoturbidimetry, and enzyme-linked immunosorbent assay (ELISA).

10. The immunological detection method according to any one of claims 1 to 3, which does not detect a monomer of type I collagen N-terminal propeptide derived from a renal disease patient.

11. An immunological detection kit for trimeric type I collagen N-terminal propeptide in a biological sample, comprising:
a first antibody that binds to a specific portion of a pro-α1 chain in the trimeric type I collagen N-terminal propeptide; and
a second antibody that binds to a specific portion of a pro-α2 chain in the trimeric type I collagen N-terminal propeptide.

12. The immunological detection kit according to claim 11, wherein the first antibody is labeled with a labeling substance;
the biological sample and the first antibody are contacted to form a first complex;
the first complex and the second antibody are contacted to form a second complex; and
a signal derived from the labeling substance is detected.

13. The immunological detection kit according to claim 11, wherein the second antibody is labeled with a labeling substance;
the biological sample and the second antibody are contacted to form a first complex;
the first complex and the first antibody are contacted to form a second complex; and
a signal derived from the labeling substance is detected.

14. The immunological detection kit according to any one of claims 11 to 13, wherein a primary structure of the specific portion of the pro-α1 chain in the trimeric type I collagen N-terminal propeptide comprises a sequence represented by SEQ ID NO: 1 or SEQ ID NO: 2:
| | |
|---|---|
| SEQ ID NO: 1 | QEEGQVEGQD |
| SEQ ID NO: 2 | PDGSESPTDQETT. |

15. The immunological detection kit according to any one of claims 11 to 13, wherein a primary structure of the specific portion of the pro-α2 chain in the trimeric type I collagen N-terminal propeptide comprises a sequence represented by SEQ ID NO: 3:
| | |
|---|---|
| SEQ ID NO: 3 | QSLQEETVRK. |

16. The immunological detection kit according to any one of claims 11 to 13, wherein the first antibody reacts with a portion of a polypeptide having a sequence represented by SEQ ID NO: 4:
| | |
|---|---|
| SEQ ID NO: 4 | JSLQEETVRK. |

17. The immunological detection kit according to claim 12 or 13, wherein the biological sample is at least one selected from the group consisting of blood, plasma, and serum.

18. The immunological detection kit according to any one of claims 11 to 13, wherein at least one of the first antibody and the second antibody is a monoclonal antibody.

19. The immunological detection kit according to any one of claims 11 to 13, which utilizes any one immunological detection method selected from the group consisting of an electrochemiluminescence method, latex agglutination immunoturbidimetry, and enzyme-linked immunosorbent assay (ELISA).

20. The immunological detection kit according to any one of claims 11 to 13, which does not detect a monomer of type I collagen N-terminal propeptide derived from a renal disease patient.
